# FASCICULE DE BREVET EUROPEEN

(11) **EP 3 356 540 B1**
(45) Date de publication et mention de la délivrance du brevet: **03.06.2020**
(21) Numéro de dépôt: 16785252.4
(22) Date de dépôt: 29.09.2016
(51) Int. Cl.: C12P 13/12

(54) **PROCÉDÉ DE PRODUCTION DE L-MÉTHIONINE**
VERFAHREN ZUR HERSTELLUNG VON L-METHIONIN
PROCESS FOR PRODUCING L-METHIONINE

(30) Priorité: 30.09.2015 FR 1559273
(43) Date de publication de la demande: 08.08.2018
(73) Titulaire: Arkema France, 92700 Colombes (FR)
(72) Inventeur: FREMY, Georges, 64390 Sauveterre De Bearn (FR); MASSELIN, Arnaud, 35400 Saint Malo (FR)
(86) Numéro de dépôt international: PCT/FR2016/052481
(87) Numéro de publication internationale: WO 2017/055754

(56) Documents cités:
- EP-A1- 0 649 837
- EP-A2- 2 402 453
- WO-A1-2008/013432
- WO-A1-2013/029690
- WO-A2-2007/011939
- BOLTEN C J ET AL: "Towards methionine overproduction in Corynebacterium glutamicum--methanethiol and dimethyldisulfide as reduced sulfur sources", JOURNAL OF MICROBIOLOGY AND BIOTECHNOLOGY, KOREAN SOCIETY FOR APPLIED MICROBIOLOGY, SEOUL, KR, vol. 20, no. 8, 1 août 2010 (2010-08-01), pages 1196-1203, XP002754158, ISSN: 1017-7825, DOI: 10.4014/JMB.1002.02018 [extrait le 2010-06-11]

## Description

La présente invention concerne un procédé de production de L-méthionine par réaction enzymatique entre un précurseur de L-méthionine, du disulfure de diméthyle (DMDS) et un composé organique réducteur. Elle concerne également un procédé en deux étapes de production de L-méthionine par réaction enzymatique entre un précurseur de L-méthionine et du méthylmercaptan, ce dernier étant obtenu par hydrogénolyse enzymatique du DMDS.

La méthionine est l'un des acides aminés essentiels du corps humain et est largement utilisée comme additif pour l'alimentation animale. Elle est en outre utilisée en tant que matière première pour des produits pharmaceutiques. La méthionine agit comme un précurseur de composés tels que la choline (lécithine) et créatine. C'est également une matière première de synthèse pour la cystéine et la taurine.

La S-adénosyl-L-méthionine (SAM) est un dérivé de la L-méthionine et est impliquée dans la synthèse de divers neurotransmetteurs dans le cerveau. La L-méthionine, et/ou la SAM, inhibe l'accumulation de lipides dans l'organisme et améliore la circulation sanguine dans le cerveau, le cœur et les reins. La L-méthionine peut également être utilisée pour favoriser la digestion, la désintoxication et l'excrétion de substances toxiques ou de métaux lourds tels que le plomb. Elle a un effet anti-inflammatoire sur les os et les maladies articulaire et c'est aussi un nutriment essentiel pour les cheveux, empêchant ainsi leur chute prématurée et non désirée.

La méthionine est déjà connue pour être préparée industriellement par des voies chimiques à partir de matières premières issues de la pétrochimie, comme décrit par exemple dans les documents FR2903690, WO2008006977, US2009318715, US5990349, JP19660043158 et WO9408957. En dehors du fait que ces méthodes de préparation ne s'inscrivent pas dans un processus de développement durable, ces voies chimiques présentent l'inconvénient de produire un mélange à part égale des deux énantiomères L et D.

Des synthèses totalement biologiques par fermentation bactérienne ont été proposées dans la littérature avec l'avantage de ne produire que l'énantiomère L de la méthionine, comme décrit par exemple dans les demandes internationales WO07077041, WO09043372, WO10020290 et WO10020681. Néanmoins, l'absence de réalisation industrielle à grande échelle à ce jour, laisse supposer que les performances et/ou les coûts de revient de ces procédés restent insuffisants.

Des procédés mixtes chimiques/biologiques viennent d'être industrialisés avec succès conjointement par la société CJ Cheil-Jedang et la Demanderesse, dans lesquels un précurseur de la L-méthionine est produit par fermentation bactérienne et réagit ensuite enzymatiquement avec du méthylmercaptan pour produire de la L-méthionine exclusivement (cf. WO2008013432 et/ou WO2013029690). Ces procédés bien que très performants nécessitent la synthèse sur site de méthylmercaptan, qui lui-même nécessite la synthèse d'hydrogène par reformage à la vapeur de méthane, la synthèse d'hydrogène sulfuré par hydrogénation du soufre et sa synthèse à partir de méthanol et d'hydrogène sulfuré, c'est-à-dire des équipements très importants peu compatibles avec une extrapolation industrielle plus modeste en terme de production annuelle que celle déjà existante.

Il subsiste donc un besoin pour produire de la L-méthionine par un procédé mixte dans lequel les équipements requis pour la synthèse de méthylmercaptan seront moindres que pour une synthèse à partir d'hydrogène, d'hydrogène sulfuré et de méthanol. C'est dans cette optique que s'inscrit la présente invention.

La présente invention propose en effet de remplacer le méthylmercaptan dans le procédé résumé ci-dessous (WO2008013432 et/ou WO2013029690), par du disulfure de diméthyle (DMDS) :

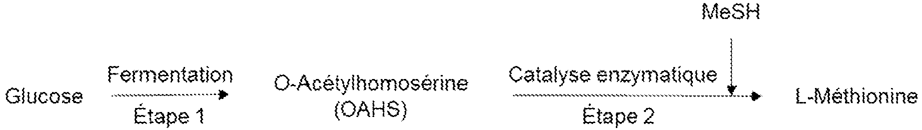

Le méthylmercaptan (MeSH) est ici directement utilisé dans la deuxième étape. La présente invention propose de substituer le méthylmercaptan par le produit d'hydrogénolyse enzymatique du disulfure de diméthyle dans une étape préalable ou combiner l'ensemble en une réaction « one pot », où le glucose et le DMDS produisent la L-méthionine.

Concernant la synthèse de méthylmercaptan à partir de disulfure de diméthyle, on peut trouver dans l'art antérieur les éléments suivants.

La demande de brevet EP0649837 propose un procédé de synthèse de méthylmercaptan par hydrogénolyse catalytique, avec des sulfures de métaux de transition, du disulfure de diméthyle avec de l'hydrogène. Ce procédé, bien que performant, nécessite des températures relativement élevées de l'ordre de 200°C pour obtenir des productivités intéressantes industriellement.

L'homme de l'art sait également qu'il est possible de préparer du méthylmercaptan par acidification d'une solution aqueuse de méthylmercaptide de sodium (CH₃SNa). Cette méthode présente l'inconvénient majeur de produire de grandes quantités de sels, tels que le chlorure de sodium ou le sulfate de sodium, selon que l'on utilise l'acide chlorhydrique ou l'acide sulfurique. Ces solutions aqueuses salines sont souvent très difficiles à traiter et les traces de produits malodorants qui subsistent font que cette méthode est difficilement envisageable sur le plan industriel.

Il a maintenant été trouvé que l'on pouvait préparer le méthylmercaptan par réduction enzymatique du disulfure de diméthyle (DMDS) lors d'une étape préalable à la synthèse de la L-méthionine et il a également été trouvé, de façon surprenante, que l'on pouvait réaliser cette réduction enzymatique du DMDS pendant la synthèse de la L-méthionine.

Ainsi, la présente invention a-t-elle pour objet un procédé de préparation de L-méthionine similaire à celui proposé dans les demandes internationales WO2008013432 et/ou WO2013029690 et qui permet de s'affranchir de, voire tout au moins de diminuer, la manutention du méthylmercaptan, en générant ledit méthylmercaptan dans une réaction de catalyse enzymatique du DMDS, juste avant l'utilisation dudit méthylmercaptan dans la synthèse de méthionine ou en générant ledit méthylmercaptan dans une réaction de catalyse enzymatique du DMDS in situ dans le réacteur de synthèse de L-méthionine.

Plus particulièrement, la présente invention a comme premier objet le procédé de préparation de L-méthionine, comprenant au moins les étapes de :
a) préparation d'un mélange comprenant :
   1) du disulfure de diméthyle (DMDS),
   2) une quantité catalytique d'acide aminé porteur d'un groupement thiol ou d'un peptide à groupement thiol,
   3) une quantité catalytique d'enzyme catalysant la réaction de réduction de pont disulfure dudit acide aminé porteur d'un groupement thiol ou dudit peptide à groupement thiol,
   4) un composé organique réducteur en quantité stœchiométrique par rapport au disulfure, en particulier le DMDS,
   5) une quantité catalytique d'enzyme catalysant la réaction de déshydrogénation du composé organique réducteur concerné,
   6) une quantité catalytique d'un cofacteur commun aux deux enzymes du système catalytique (déshydrogénase et réductase),
b) conduite de la réaction enzymatique pour former le méthylmercaptan (CH₃-SH),
c) ajout d'un précurseur de la L-méthionine et conversion dudit précurseur avec le méthylmercaptan formé à l'étape b), et
d) récupération et éventuellement purification de la L-méthionine formée.

Les composants de l'étape a) ci-dessus peuvent être ajoutés dans différents ordres (l'ordre d'addition dans l'étape a) n'est pas restrictif). Dans un mode de réalisation de l'invention, l'acide aminé porteur d'un groupement thiol et/ou le peptide porteur d'un groupement thiol peut être sous la forme du disulfure dudit acide aminé et/ou dudit peptide respectivement, par exemple glutathion sous forme de disulfure de glutathion.

D'une manière générale, l'enzyme catalysant la réduction du pont disulfure créé entre deux équivalents dudit acide aminé porteur d'un groupement thiol ou dudit peptide à groupement thiol est une enzyme réductase. Le terme « réductase » est utilisé dans la suite de la description pour l'explication de la présente invention. De manière similaire, l'enzyme catalysant la déshydrogénation du composé organique réducteur impliqué dans l'étape b) est généralement dénommée enzyme déshydrogénase, le terme « déshydrogénase » étant choisi dans la suite de la description pour l'explication de la présente invention.

Parmi les cofacteurs communs aux deux enzymes catalysant la réduction et la déshydrogénation (la réductase et la déshydrogénase), on peut citer à titre d'exemples non limitatifs les cofacteurs flaviniques, et les cofacteurs nicotiniques. On préfère utiliser les cofacteurs nicotiniques et plus particulièrement la Nicotinamide Adénine Dinucléotide (NAD), ou mieux encore la Nicotinamide Adénine Dinucléotide Phosphate (NADPH). Les cofacteurs listés ci-dessus sont avantageusement utilisés sous leurs formes réduites (par exemple NADPH, H+) et/ou leurs formes oxydées (par exemple NADP+), c'est-à-dire qu'ils peuvent être ajoutés sous ces formes réduites et/ou oxydées, dans le milieu réactionnel.

L'organisation et l'ordre des ajouts des composants 1) à 6) dans l'étape a) peuvent être réalisés de différentes manières. La réaction enzymatique de l'étape b) est déclenchée par l'ajout d'un des composants du système catalytique du mélange de l'étape a) : soit une enzyme, soit un des composés ajoutés en quantité stœchiométrique (disulfure ou composé organique réducteur) soit un des composés ajoutés en quantité catalytique (acide aminé porteur d'un groupement thiol ou peptide à groupement thiol ou du disulfure correspondant audit thiol ou audit peptide ou bien encore le cofacteur).

Ainsi et selon un mode de réalisation de la présente invention le procédé de préparation de L-méthionine, comprend au moins les étapes de :
a') préparation d'un mélange comprenant :
   - du disulfure de diméthyle (DMDS),
   - une quantité catalytique d'acide aminé porteur d'un groupement thiol ou d'un peptide à groupement thiol,
   - une quantité catalytique d'enzyme réductase correspondant audit acide aminé porteur d'un groupement thiol ou audit peptide à groupement thiol,
   - une quantité catalytique de NADPH,
b') ajout d'un composé organique réducteur en quantité stœchiométrique par rapport au disulfure de diméthyle) avec une quantité catalytique de l'enzyme déshydrogénase correspondante,
c') conduite de la réaction enzymatique pour former le méthylmercaptan (CH₃-SH),
d') conversion d'un précurseur de la L-méthionine avec le méthylmercaptan formé à l'étape c'), et
e') récupération et éventuellement purification de la L-méthionine formée.

Selon le procédé de l'invention, le méthylmercaptan, généralement formé à l'état gazeux, est alors directement mis en contact avec un précurseur de méthionine comme décrit plus loin.

Le procédé de synthèse de L-méthionine selon l'invention est tout d'abord basé sur la réduction enzymatique du disulfure de diméthyle avec un composé organique réducteur, qui est un donneur d'hydrogène ainsi qu'il sera défini plus loin, selon la réaction suivante, en utilisant du glucose comme composé organique réducteur (donneur d'hydrogène) :

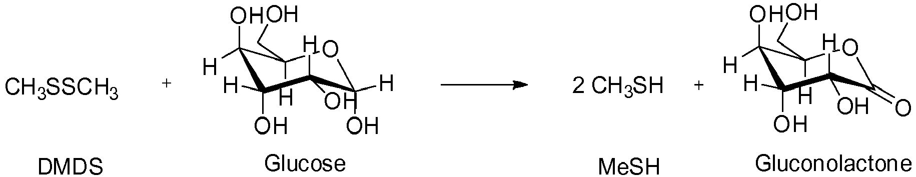

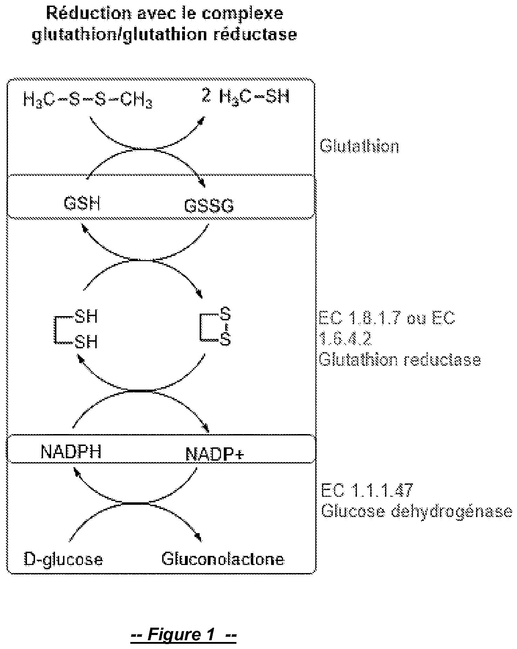

Il a maintenant été découvert que cette réaction est facilement catalysée par le système enzymatique mettant en œuvre un acide aminé à groupement thiol ou un peptide à groupement thiol, par exemple le glutathion, sous forme de complexe (acide aminé ou peptide)/enzyme réductase correspondante, régénéré par le composé organique donneur d'hydrogène, comme décrit à la Figure 1 annexée.

Ainsi selon l'illustration de la Figure 1, le peptide (« glutathion » représenté) réduit le disulfure (« DMDS » représenté) en mercaptan (« méthylmercaptan » représenté), en se transformant en peptide à pont disulfure (« disulfure de glutathion » représenté). L'enzyme réductase (« glutathion réductase » représentée, EC 1.8.1.7 ou EC 1.6.4.2) régénère le peptide (glutathion), tout en oxydant le cofacteur (« NADPH, H+ » représenté). La forme oxydée (« NADP+ » représenté) est alors réduite à l'aide d'un complexe enzymatique oxydo-réducteur, dit « de recyclage », bien connu de l'homme de métier et comprenant l'enzyme déshydrogénase impliquée (« glucose déshydrogénase » représentée, avec l'exemple de numéro de classification enzymatique EC 1.1.1.47) et la molécule organique réductrice (« glucose » représenté). La forme oxydée du composé organique réducteur est alors obtenue (« gluconolactone » représentée).

Plus particulièrement, le peptide (exemple représenté le glutathion) réduit le disulfure de diméthyle en méthylmercaptan en se transformant en peptide à pont disulfure (disulfure de glutathion représenté). L'enzyme réductase (« glutathion réductase » représentée, EC 1.8.1.7 ou EC 1.6.4.2) régénère le peptide (glutathion) et cette même enzyme est régénérée par un complexe enzymatique oxydo-réducteur bien connu de l'homme du métier, par exemple le complexe NADPH/NADP+ (Nicotine adénine dinucléotide phosphate (forme réduite et forme oxydée)). À son tour NADP+ est régénéré en NADPH par l'intermédiaire de l'enzyme déshydrogénase correspondante au composé organique réducteur employé (ici la «glucose déshydrogénase» EC 1.1.1.47) grâce audit composé organique réducteur (glucose représenté) qui fournit de l'hydrogène (donneur d'hydrogène) en se transformant en sa forme oxydée (ici la gluconolactone).

Selon un mode de réalisation tout particulièrement adapté, le système glutathion/disulfure de glutathion associé à l'enzyme glutathion-réductase permet selon la présente invention de réduire le DMDS en méthylmercaptan.

Le glutathion est un tripeptide largement utilisé en biologie. Cette espèce sous forme réduite (glutathion) ou oxydée (disulfure de glutathion) forme un couple d'oxydoréduction important dans les cellules. Ainsi le glutathion est vital pour supprimer les métaux lourds des organismes. Ainsi par exemple la demande WO05107723 décrit une formulation où le glutathion est utilisé pour former une préparation chélatante, le brevet US4657856 enseigne que le glutathion permet également de détruire les peroxydes comme l'H₂O₂ en H₂O via la glutathion peroxydase. Enfin le glutathion permet également de réduire les ponts disulfures présents dans les protéines (Rona Chandrawati, « Triggered Cargo Release by Encapsulated Enzymatic Catalysis in Capsosomes », Nano Lett., (2011), vol. 11, 4958-4963).

Selon le procédé de l'invention, une quantité catalytique d'acide aminé porteur d'un groupement thiol ou d'un peptide à groupement thiol, est mise en œuvre pour la production de méthylmercaptan à partir de disulfure de diméthyle.

Parmi les acides aminés porteurs de groupement thiol utilisables dans le procédé de la présente invention, on peut citer, à titre d'exemples non limitatifs la cystéine et l'homocystéine. Les systèmes enzymatiques oxydo-réducteurs utilisés pouvant régénérer le cycle catalytique de la même façon, sont dans ces cas le système cystéine/cystine-réductase EC 1.8.1.6, et homocystéine/homocystéine-réductase.

Il peut être avantageux d'utiliser l'homocystéine car cet acide aminé peut être préparé à partir d'OAHS (précurseur de la L-méthionine), de sulfure d'hydrogène (H₂S) et de l'enzyme de la méthionine, c'est-à-dire de l'enzyme catalysant la réaction menant à la méthionine. Ainsi, une très faible quantité d'H₂S dans le milieu réactionnel crée, *in situ,* le cycle équivalent à celui du glutathion.

Parmi les peptides porteurs de groupement thiol utilisables dans le procédé de la présente invention, on peut citer, à titre d'exemples non limitatifs le glutathion et la thiorédoxine. Le système glutathion/glutathion réductase, décrit précédemment, peut ainsi être remplacé par le système thiorédoxine (CAS no. 52500-60-4)/thiorédoxine réductase (EC 1.8.1.9 ou EC 1.6.4.5).

Le glutathion et le système glutathion/glutathion réductase sont tout particulièrement préférés pour la présente invention, en raison de la facilité d'approvisionnement et des coûts de ces composés.

Parmi les composés organiques réducteurs qui peuvent être utilisés dans le cadre de la présente invention, les composés donneurs d'hydrogène sont tout particulièrement préférés, et parmi ceux-ci, les composés tout à fait adaptés sont les composés organiques réducteurs donneurs d'hydrogène porteurs de fonction hydroxyle, tels que les alcools, les polyols, les sucres et autres.

L'enzyme utilisée est une enzyme apte à déshydrogéner le composé porteur d'hydrogène, par exemple une alcool-déshydrogénase. Le glucose est un sucre tout particulièrement bien adapté pour être mis en œuvre dans le procédé de la présente invention, avec la glucose-déshydrogénase, pour donner la gluconolactone.

Dans le procédé selon l'invention, dans le cas où la réduction enzymatique du DMDS se fait dans un réacteur séparé de la synthèse de la L-méthionine, seul le glucose est utilisé en quantité stœchiométrique, tous les autres composants (glutathion, cofacteur (par exemple NADPH) et les deux enzymes) sont utilisés en quantité catalytique. Dans le cas où la réaction de réduction enzymatique du DMDS se fait avec la synthèse de la L-méthionine dans un seul réacteur dit « one pot », le précurseur de la L-méthionine est également ajouté en quantité stœchiométrique, tandis que les réactifs complémentaires à cette synthèse tels que le phosphate de pyridoxal (PLP) et l'enzyme spécifique à cette réaction sont ajoutés en quantités catalytiques.

Les concentrations en phosphate de pyridoxal et en enzyme spécifique au précurseur préférées sont celles que l'on peut trouver dans les demandes internationales WO2008013432 et/ou WO2013029690.

Les avantages que procure la synthèse par catalyse enzymatique de méthylmercaptan à partir de disulfure de diméthyle sont nombreux, que ce soit dans le cas des deux étapes successives ou du procédé « one pot ». Parmi ces avantages, on peut citer la possibilité de travailler en solution aqueuse ou hydro-organique, dans des conditions très douces de température et de pression et dans des conditions de pH proches de la neutralité. Toutes ces conditions sont typiques d'un procédé dit « vert » ou « durable », et sont tous à fait compatibles avec la préparation de L-méthionine, telle que décrite dans les demandes internationales WO2008013432 et/ou WO2013029690.

Un autre avantage lorsque le procédé met en œuvre du disulfure de diméthyle est que le méthylmercaptan produit, qui est à l'état gazeux dans les conditions de réaction, sort du milieu réactionnel au fur et à mesure de sa formation. Le méthylmercaptan peut donc être directement utilisé à la sortie du réacteur dans la synthèse de la L-méthionine, comme décrit par exemple dans WO2008013432 et/ou WO2013029690, c'est-à-dire à partir par exemple d'O-acétylhomosérine ou d'O-succinylhomosérine et des enzymes telles que l'O-acétylhomosérine-sulfhydrylase ou l'O-succinylhomosérine-sulfhydrylase respectivement.

Le méthylmercaptan peut aussi être facilement liquéfié par cryogénie par exemple si on souhaite l'isoler. On peut éventuellement accélérer son départ du milieu réactionnel en introduisant par bullage un léger débit de gaz inerte, avantageusement l'azote.

Les gaz de sortie contenant de l'azote et du méthylmercaptan peuvent, si on le souhaite et si nécessaire, être recyclés dans le premier réacteur (réduction enzymatique du DMDS) après passage dans le second réacteur (synthèse de L-méthionine) si le méthylmercaptan n'a pas été complètement converti en L-méthionine. Le procédé selon l'invention décrit donc un procédé de synthèse de L-méthionine en deux étapes enzymatiques successives à partir d'un précurseur de L-méthionine et de DMDS.

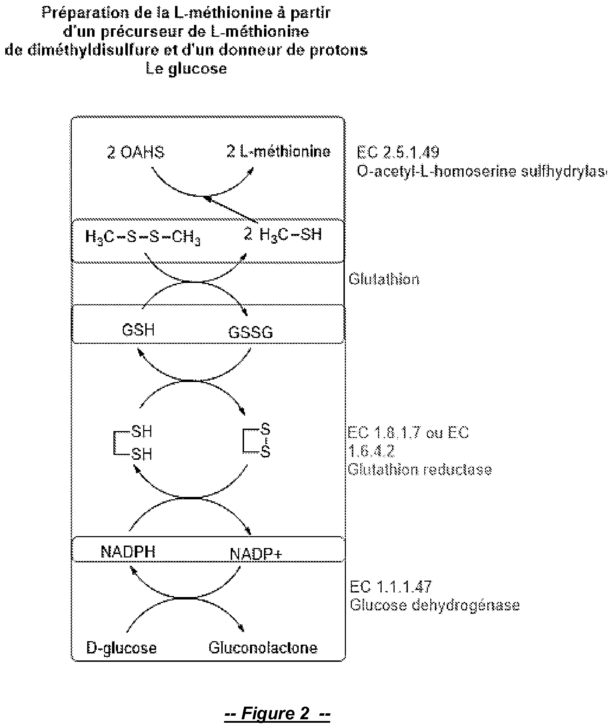

Il est également possible de réaliser la synthèse de L-méthionine dans un seul et même réacteur. Dans ce cas on ajoute au système de réduction enzymatique du DMDS (étape a) ci-dessus) tous les réactifs nécessaires à la synthèse de la L-méthionine et on ferme le réacteur pour éviter le départ du méthylmercaptan formé par réduction enzymatique *in situ* du DMDS. Le méthylmercaptan réagit alors avec le précurseur de la L-méthionine pour donner la L-méthionine. Le procédé selon la présente invention décrit ainsi un procédé de synthèse directe de L-méthionine à partir d'un précurseur de L-méthionine et de DMDS, comme illustré par la Figure 2 annexée, où la synthèse à partir de OAHS, de DMDS et glucose.

Le disulfure de diméthyle (DMDS) peut être produit sur un autre site à partir de méthylmercaptan et d'un oxydant tels que l'oxygène, le soufre ou l'eau oxygénée par exemple, ou encore à partir de sulfate de diméthyle et de disulfure de sodium. Le DMDS peut aussi provenir d'une source de « DiSulfide Oils » (DSO) purifiée par exemple par distillation réactive comme décrit dans la demande WO2014033399.

La réduction par catalyse enzymatique de DMDS peut être considérée comme un procédé permettant d'éviter le transport de méthylmercaptan de son site de production par les voies existantes industrielles, vers son site d'utilisation, s'ils sont différents. En effet, le méthylmercaptan est un gaz à température ambiante, toxique et fortement malodorant ce qui complique énormément son transport déjà très réglementé contrairement au DMDS. Ainsi le DMDS peut donc être utilisé pour produire du méthylmercaptan directement sur le site d'utilisation de ce dernier dans la synthèse de la L-méthionine, réduisant ainsi encore les inconvénients liés à la toxicité et à l'odeur de ce produit, ainsi que les risques industriels qui y sont attachés.

Dans le cas du procédé de synthèse en deux étapes successives, le DMDS étant consommé dans la réaction et le méthylmercaptan sortant du milieu réactionnel au fur et à mesure de sa formation, seul le produit de déshydrogénation du composé organique réducteur, par exemple la gluconolactone, s'accumule dans le milieu réactionnel, dans l'hypothèse d'une alimentation en continu de glucose et de DMDS. Lorsque la concentration en gluconolactone dépasse la saturation dans les conditions de réaction, cette dernière va précipiter et pourra alors être isolée du milieu réactionnel par tout moyen connu de l'homme du métier.

La gluconolactone peut avoir plusieurs utilisations. Elle est par exemple utilisée en tant qu'additif alimentaire connu sous le sigle E575. La gluconolactone s'hydrolyse en milieux aqueux acides pour former de l'acide gluconique également utilisé comme additif alimentaire (E574). La gluconolactone est également utilisée pour la production de tofu (cf. CN103053703) pour l'industrie alimentaire.

La gluconolactone peut notamment et avantageusement, dans le sens où elle représente le « déchet » du procédé selon la présente invention, remplacer le glucose dans une éventuelle réaction de fermentation pour produire soit du bioéthanol soit toute autre molécule issue de fermentation de sucre ou d'amidon.

Certaines bactéries peuvent en effet utiliser en fermentation la gluconolactone comme source de carbone, comme décrit par J. P. van Dijken, « Novel pathway for alcoholic fermentation of gluconolactone in the yeast Saccharomyces bulderi », J. Bacteriol., (2002), Vol. 184(3), 672-678.

Un intérêt évident de la gluconolactone dans le procédé selon l'invention est de la recycler dans la synthèse du précurseur de la L-méthionine. En effet cette synthèse étant une fermentation bactérienne utilisant du glucose, la gluconolactone pourrait aisément remplacer une partie de ce glucose. Ce recyclage peut dans ces conditions représenter un avantage économique très significatif.

Même dans le cas où la réaction se fait dans les conditions « one pot » définies plus haut, et la gluconolactone étant beaucoup plus soluble que la L-méthionine, il est aisé de la séparer du milieu réactionnel selon des techniques classiques et bien connues de l'homme du métier.

D'autres sucres encore peuvent être utilisés dans le procédé de l'invention, et par exemple il est possible de remplacer le système glucose/gluconolactone/ glucose-déshydrogénase par le système suivant : glucose-6-phosphate/ 6-phosphoglucono-δ-lactone/Glucose-6-phosphate déshydrogénase (EC 1.1.1.49).

Il est également possible dans le procédé de l'invention d'utiliser un alcool en remplacement du sucre, et ainsi utiliser à la place du système glucose/gluconolactone/glucose déshydrogénase, le système général suivant : alcool/cétone ou aldéhyde / alcool-déshydrogénase (EC 1.1.1) et plus particulièrement le système isopropanol/acétone/isopropanol-déshydrogénase (EC 1.1.1.80).

En effet, ce système permet d'obtenir, à partir de DMDS et d'isopropanol, un mélange constitué de méthylmercaptan (MeSH) et d'acétone qui sort du milieu réactionnel (donc pas d'accumulation d'aucun produit). Le MeSH et l'acétone peuvent être facilement séparés par une simple distillation si on le désire.

Selon un mode de réalisation, le procédé selon l'invention comprend la préparation par réduction enzymatique du DMDS, puis réaction du méthylmercaptan formé avec un précurseur de L-méthionine pour donner la L-méthionine. Dans ce cas, le procédé selon l'invention comprend au moins les étapes suivantes :
Étape 1 : préparation d'un précurseur de la L-méthionine, par exemple par fermentation bactérienne de glucose (cf. WO2008013432 et/ou WO2013029690),
Étape 2 : réduction enzymatique du DMDS dans un réacteur R1 avec formation de méthylmercaptan sortant dudit réacteur R1 (correspondant aux étapes a') à c') ci-dessus),
Étape 3 : synthèse enzymatique de la L-méthionine dans un réacteur R2 avec le précurseur de l'Étape 1 et le méthylmercaptan de l'Étape 2 (correspondant à l'étape d') ci-dessus),
Étape 4 (optionnelle) : recyclage de la gluconolactone formée à l'Étape 3 dans l'Étape 1,
Étape 5 : récupération et éventuellement purification de la L-méthionine formée (correspondant à l'étape e') ci-dessus).

Pour l'Étape 1, on trouvera le domaine de conditions utilisables dans les brevets suivants (cf. WO2008013432 et/ou WO2013029690).

Pour l'Étape 2, la température de la réaction est comprise dans un domaine allant de 10°C à 50°C, de préférence entre 15°C et 45°C, de préférence encore entre 20°C et 40°C.

Le pH de la réaction peut être compris entre 6 et 8, de préférence entre 6,5 et 7,5. Le pH du milieu réactionnel peut être ajusté au moyen d'un tampon. De façon tout à fait préférée, on choisira, par exemple, le pH du tampon phosphate à 0,1 mol.L⁻¹ à 7,3.

La pression utilisée pour la réaction peut aller d'une pression réduite par rapport à la pression atmosphérique à plusieurs bars (plusieurs centaines de kPa), selon les réactifs utilisés et le matériel utilisé. Une pression réduite peut en effet permettre un dégazage plus rapide du méthylmercaptan formé mais présente l'inconvénient d'augmenter les pressions de vapeur saturante de l'eau et du DMDS, polluant un peu plus le méthylmercaptan formé. De façon préférée, on utilisera une pression allant de la pression atmosphérique à 20 bars (2 MPa) et de façon encore plus préférée on travaillera sous une pression allant de la pression atmosphérique à 3 bars (300 kPa).

Pour l'Étape 3, on se référera à la demande internationale WO2013029690 pour les conditions idéales avec la différence possible d'introduire un débit d'azote dans le réacteur R1 pour arriver dans le réacteur R2 et recycler ces gaz du réacteur R2 vers le réacteur R1 à la pression voulue si le méthylmercaptan n'a pas totalement réagi dans le réacteur R2.

Selon un autre mode de réalisation (autre variante), le procédé selon la présente invention est réalisé dans un seul et même réacteur (« one pot »), et dans ce cas comprend au moins les étapes suivantes :
Étape 1' : préparation d'un précurseur de la L-méthionine par fermentation bactérienne de glucose par exemple (similaire à l'Étape 1 ci-dessus),
Étape 2' : réduction enzymatique du DMDS dans un réacteur R1 avec formation *in situ* de méthylmercaptan et synthèse conjointe enzymatique de la L-méthionine dans le même réacteur avec le précurseur obtenu à l'Étape 1',
Étape 3' (optionnelle) : recyclage de la gluconolactone formée à l'Étape 2' dans l'Étape 1', et
Étape 4' : récupération et éventuellement purification de la L-méthionine formée.

Pour l'Étape 1', on trouvera le domaine de conditions utilisables dans les demandes internationales WO2008013432 et/ou WO2013029690.

Pour l'Étape 2', les conditions opératoires sont les suivantes.

La température de la réaction est comprise dans un domaine allant de 10°C à 50°C, de préférence de 15°C à 45°C, de préférence encore de 20°C à 40°C.

Le pH de la réaction est avantageusement compris entre 6 et 8, de préférence entre 6,2 et 7,5. De façon tout à fait préférée, la réaction est effectuée au pH du tampon phosphate à 0,2 mol.L⁻¹ et égal à 7,0.

De façon préférée, le procédé est réalisé à une pression allant de la pression atmosphérique à 20 bars (2 MPa) et de façon encore plus préférée, de la pression atmosphérique à 3 bars (300 kPa).

Le ratio molaire DMDS/précurseur de L-méthionine est compris entre 0,1 et 10, généralement entre 0,5 et 5, et de préférence le ratio molaire est la stœchiométrie (ratio molaire = 0,5) mais peut être supérieur si cela s'avère bénéfique pour la cinétique de la réaction.

Dans l'une ou l'autre des variantes du procédé selon l'invention, celui-ci peut être réalisé en batch ou en continu, dans un réacteur en verre ou en métal suivant les conditions opératoires retenues et les réactifs utilisés.

Dans l'une ou l'autre des variantes du procédé selon l'invention, le ratio molaire composé organique réducteur/DMDS idéal est la stœchiométrie (ratio molaire = 1) mais peut varier de 0,01 à 100 si l'homme du métier y trouve un intérêt quelconque telle qu'une addition en continu du DMDS alors que le composé réducteur est introduit dès le départ dans le réacteur. De façon préférée ce ratio molaire est choisi entre 0,5 et 5 globalement sur l'ensemble de la réaction.

Les éléments présents en quantité catalytique dans le mélange préparé à l'étape a) ci-dessus (acide aminé porteur d'un groupement thiol ou d'un peptide à groupement thiol ou encore le disulfure correspondant audit acide aminé ou le disulfure correspondant audit peptide, enzyme réductase, enzyme déshydrogénase, cofacteur, par exemple NADPH) sont aisément accessibles dans le commerce ou peuvent être préparés selon des techniques bien connues de l'homme du métier. Ces différents éléments peuvent se présenter sous forme solide ou liquide et peuvent très avantageusement être mis en solution dans l'eau pour être mis en œuvre dans le procédé de l'invention. Les enzymes utilisées peuvent également être greffées sur support (cas des enzymes supportées).

La solution aqueuse de complexe enzymatique comprenant l'acide aminé ou le peptide peut également être reconstituée par les méthodes connues par l'homme de métier, par exemple par perméabilisation de cellules qui contiendraient ces éléments. Cette solution aqueuse dont une composition est donnée dans l'exemple 1 suivant peut être utilisée dans des teneurs en masse comprises entre 0,01% et 20% par rapport au poids total du milieu réactionnel. De façon préférée on utilisera une teneur comprise entre 0,5% et 10%.

On comprendra mieux l'invention avec les exemples suivants non limitatifs par rapport à la portée de l'invention.

### EXEMPLE 1 : Procédé en 2 étapes successives

Dans un réacteur R1 contenant 150 mL de tampon phosphate à 0,1 mol/L à pH 7,30, sont introduits 10 mL de complexe enzymatique au glutathion (Aldrich) et 19,2 g (0,1 mol) de glucose. La solution de complexe enzymatique contient : 185 mg (0,6 mmol) de glutathion, 200 U de glutathion-réductase, 50 mg (0,06 mmol) de NADPH et 200 U de glucose-déshydrogénase. Le milieu réactionnel est porté à 25°C sous agitation mécanique. Un premier prélèvement est effectué t = 0. Par la suite, le disulfure de diméthyle (9,4 g, 0,1 mol) est placé dans une burette et ajouté au goutte à goutte dans le réacteur, la réaction commence. Un courant d'azote est placé dans le réacteur.

Une analyse en chromatographie en phase gazeuse des gaz sortant du réacteur montre quasi essentiellement la présence d'azote et de méthylmercaptan (quelques traces d'eau). Ces gaz de sortie sont envoyés dans le réacteur R2. Le DMDS est introduit en 6 heures dans le réacteur R1. Une analyse finale en chromatographie en phase gazeuse du milieu réactionnel du réacteur R1 confirme l'absence de DMDS, et une analyse par UPLC/masse montre des traces de glucose et la présence quasi exclusive de gluconolactone (traces d'acide gluconique).

En parallèle, dans le second réacteur R2 contenant 75 mL de tampon phosphate 0,1 mol.L⁻¹ à pH 6,60, sont introduits 5 g d'O-acétyl-L-homosérine (OAHS) (l'O-acétylhomosérine a été synthétisée à partir de L-homosérine et d'anhydride acétique selon Sadamu Nagai, « Synthesis of O-acetyl-L-homoserine », Academic Press, (1971), vol.17, p. 423-424). La solution est portée à 35°C sous agitation mécanique.

Avant tout commencement de la réaction, un prélèvement (t = 0) de 1 mL du milieu réactionnel est effectué. Une solution de phosphate de pyridoxal (1,6 mmol, 0,4 g) et l'enzyme O-acétyl-L-homosérine-sulfhydrylase (0,6 g) sont dissous dans 10 mL d'eau puis ajoutés dans le réacteur.

Le méthylmercaptan est introduit via la réaction du réacteur R1 et poussé par un courant d'azote. La réaction commence alors. La formation de L-méthionine et la disparition de l'OAHS sont suivies par HPLC. Les gaz de sortie du réacteur R2 sont piégés dans une solution aqueuse de soude (hydroxyde de sodium) à 20%. Les analyses montrent que l'OAHS a été convertie à 52% en L-méthionine et que l'excès de DMDS a été converti en méthylmercaptan retrouvé dans le piège à soude.

### EXEMPLE 2 : Procédé « one pot »

Dans un réacteur contenant 150 mL de tampon phosphate 0,2 mol.L⁻¹ à pH 7, sont introduits 10 mL du complexe enzymatique, 6 g (33 mmol) de glucose et 5 g (31 mmol) d'O-acétyl-L-homosérine (OAHS, l'O-acétyl-L-homosérine a été synthétisée à partir de L-homosérine et d'anhydride acétique selon Sadamu Nagai, « Synthesis of O-acetyl-L-homoserine », Academic Press, (1971), vol.17, p. 423-424). La solution du complexe enzymatique contient: 185 mg (0,6 mmol) de glutathion, 200 U de glutathion-réductase, 50 mg (0,06 mmol) de NADPH, 200 U de glucose-déshydrogénase, 0,4 g (1,6 mmol) de phosphate de pyridoxal et 0,6 g de O-acétyl-L-homosérine-sulfhydrylase.

Le milieu réactionnel est porté à 27°C sous agitation mécanique. Un premier prélèvement t = 0 est effectué. Par la suite le disulfure de diméthyle (3 g, 32 mmol) est placé dans une burette et ajouté goutte à goutte dans le réacteur qui a été fermé pour éviter tout dégagement de méthylmercaptan, la réaction commence. La réaction est suivie par HPLC pour voir la disparition de l'OAHS et la formation de la L-méthionine. Au bout de 6 heures, 21% de l'OAHS ont été convertis en L-méthionine démontrant la possibilité de produire de la L-méthionine par un procédé « one-pot » à partir d'un précurseur de L-méthionine, de DMDS et d'un composé organique réducteur.

## Revendications

1. Procédé de préparation de L-méthionine, comprenant au moins les étapes de :
a) préparation d'un mélange comprenant :
1) du disulfure de diméthyle (DMDS),
2) une quantité catalytique d'acide aminé porteur d'un groupement thiol ou d'un peptide à groupement thiol,
3) une quantité catalytique d'enzyme catalysant la réaction de réduction de pont disulfure dudit acide aminé porteur d'un groupement thiol ou dudit peptide à groupement thiol,
4) un composé organique réducteur en quantité stœchiométrique par rapport au disulfure, en particulier le DMDS,
5) une quantité catalytique d'enzyme catalysant la réaction de déshydrogénation d'un composé organique réducteur concerné,
6) une quantité catalytique d'un cofacteur commun aux deux enzymes du système catalytique (déshydrogénase et réductase),
b) conduite de la réaction enzymatique pour former le méthylmercaptan (CH₃-SH),
c) ajout d'un précurseur de la L-méthionine et conversion dudit précurseur avec le méthylmercaptan formé à l'étape b), et
d) récupération et éventuellement purification de la L-méthionine formée.

2. Procédé selon la revendication 1, comprenant au moins les étapes de :
a') préparation d'un mélange comprenant :
• du disulfure de diméthyle (DMDS),
• une quantité catalytique d'acide aminé porteur d'un groupement thiol ou d'un peptide à groupement thiol,
• une quantité catalytique d'enzyme réductase correspondant audit acide aminé porteur d'un groupement thiol ou audit peptide à groupement thiol,
• une quantité catalytique de NADPH,
b') ajout d'un composé organique réducteur en quantité stœchiométrique par rapport au disulfure de diméthyle) avec une quantité catalytique de l'enzyme déshydrogénase correspondante,
c') conduite de la réaction enzymatique pour former le méthylmercaptan (CH₃-SH), d') conversion d'un précurseur de la L-méthionine avec le méthylmercaptan formé à l'étape c'), et
e') récupération et éventuellement purification de la L-méthionine formée.

3. Procédé selon la revendication 1 ou la revendication 2, dans lequel, le méthylmercaptan est directement mis en contact avec un précurseur de méthionine.

4. Procédé selon l'une quelconque des revendications précédentes, dans lequel le composé organique réducteur est un composé organique réducteur donneur d'hydrogène porteur de fonction hydroxyle, choisi parmi les alcools, les polyols, les sucres.

5. Procédé selon l'une quelconque des revendications précédentes, dans lequel le composé organique réducteur est choisi parmi le glucose, le glucose-6-phosphate et l'isopropanol.

6. Procédé selon l'une quelconque des revendications précédentes, dans lequel l'acide aminé porteur de groupement thiol ou le peptide porteur de groupement thiol est choisi parmi la cystéine, l'homocystéine, le glutathion et la thiorédoxine.

7. Procédé selon l'une quelconque des revendications précédentes, dans lequel le précurseur de la L-méthionine est choisi parmi la O-acétyl-L-homosérine et la O-succinyl-L-homosérine.

8. Procédé selon l'une quelconque des revendications précédentes, dans lequel le méthylmercaptan est directement utilisé à la sortie du réacteur dans la synthèse de la L-méthionine.

9. Procédé selon la revendication 8, comprenant au moins les étapes suivantes :
Étape 1 : préparation d'un précurseur de la L-méthionine, par exemple par fermentation bactérienne de glucose,
Étape 2 : réduction enzymatique du DMDS dans un réacteur R1 avec formation de méthylmercaptan sortant dudit réacteur R1,
Étape 3 : synthèse enzymatique de la L-méthionine dans un réacteur R2 avec le précurseur de l'Étape 1 et le méthylmercaptan de l'Étape 2,
Étape 4 (optionnelle) : recyclage de la gluconolactone formée à l'Étape 3 dans l'Étape 1,
Étape 5 : récupération et éventuellement purification de la L-méthionine formée.

10. Procédé selon l'une quelconque des revendications 1 à 7, dans lequel la synthèse de méthylmercaptan à partir de DMDS et la synthèse de L-méthionine à partir dudit méthylmercaptan est réalisée dans un seul et même réacteur.

11. Procédé selon la revendication 10, comprenant au moins les étapes suivantes :
Étape 1' : préparation d'un précurseur de la L-méthionine par fermentation bactérienne de glucose,
Étape 2' : réduction enzymatique du DMDS dans un réacteur R1 avec formation *in situ* de méthylmercaptan et synthèse conjointe enzymatique de la L-méthionine dans le même réacteur avec le précurseur obtenu à l'Étape 1',
Étape 3' (optionnelle) : recyclage de la gluconolactone formée à l'Étape 2' dans l'Étape 1', et
Étape 4' : récupération et éventuellement purification de la L-méthionine formée.

12. Procédé selon l'une quelconque des revendications précédentes, réalisé en batch ou en continu.

13. Procédé selon l'une quelconque des revendications précédentes, dans lequel le ratio molaire composé organique réducteur/DMDS idéal varie de 0,01 à 100, de façon préférée ledit ratio molaire est choisi entre 0,5 et 5, et de manière tout à fait préférée, le ratio molaire est égal à 1.

14. Procédé selon l'une quelconque des revendications précédentes, dans lequel le ratio molaire DMDS/précurseur de L-méthionine est compris entre 0,1 et 10, généralement entre 0,5 et 5, et de préférence le ratio molaire est la stœchiométrie (ratio molaire = 0,5).

15. Procédé selon l'une quelconque des revendications précédentes, dans lequel la température de la réaction est comprise dans un domaine allant de 10°C à 50°C, de préférence de 15°C à 45°C, de préférence encore de 20°C à 40°C.

## Patentansprüche

1. Verfahren zur Herstellung von L-Methionin, das mindestens folgende Schritte umfasst:
a) Herstellen einer Mischung, umfassend:
1) Dimethyldisulfid (DMDS),
2) eine katalytisch wirksame Menge von Aminosäure mit einer Thiolgruppe oder eines Peptids mit einer Thiolgruppe,
3) eine katalytisch wirksame Menge eines Enzyms, das die Reduktionsreaktion der Disulfidbrücke der Aminosäure mit einer Thiolgruppe bzw. des Peptids mit einer Thiolgruppe katalysiert,
4) eine organische reduzierend wirkende Verbindung in stöchiometrischer Menge, bezogen auf das Disulfid, insbesondere das DMDS,
5) eine katalytisch wirksame Menge eines Enzyms, das die Dehydrierungsreaktion einer betreffenden organischen reduzierend wirkenden Verbindung katalysiert,
6) eine katalytisch wirksame Menge eines Cofaktors, der den beiden Enzymen des katalytischen Systems (Dehydrogenase und Reduktase) gemein ist,
b) Durchführen der enzymatischen Reaktion zur Bildung von Methylmercaptan (CH₃-SH),
c) Zugeben einer Vorstufe von L-Methionin und Umwandeln der Vorstufe mit dem in Schritt b) gebildeten Methylmercaptan und
d) Gewinnen und gegebenenfalls Reinigen des gebildeten L-Methionins.

2. Verfahren nach Anspruch 1, das mindestens folgende Schritte umfasst:
a') Herstellen einer Mischung, umfassend:
• Dimethyldisulfid (DMDS),
• eine katalytisch wirksame Menge von Aminosäure mit einer Thiolgruppe oder eines Peptids mit einer Thiolgruppe,
• eine katalytisch wirksame Menge eines Reduktaseenzyms, das der Aminosäure mit einer Thiolgruppe bzw. dem Peptid mit einer Thiolgruppe entspricht,
• eine katalytisch wirksame Menge NADPH,
b') Zugeben einer organischen reduzierend wirkenden Verbindung in stöchiometrischer Menge, bezogen auf das Dimethyldisulfid, mit einer katalytisch wirksamen Menge des entsprechenden Dehydrogenaseenzyms,
c') Durchführen der enzymatischen Reaktion zur Bildung von Methylmercaptan (CH₃-SH),
d') Umwandeln einer Vorstufe von L-Methionin mit dem in Schritt c') gebildeten Methylmercaptan und
e') Gewinnen und gegebenenfalls Reinigen des gebildeten L-Methionins.

3. Verfahren nach Anspruch 1 oder Anspruch 2, bei dem das Methylmercaptan direkt mit einer Vorstufe von Methionin in Kontakt gebracht wird.

4. Verfahren nach einem der vorhergehenden Ansprüche, bei dem es sich bei der organischen reduzierend wirkenden Verbindung um eine hydroxylfunktionelle wasserstoffabgebende organische reduzierend wirkende Verbindung handelt, die aus Alkoholen, Polyolen und Zuckern ausgewählt wird.

5. Verfahren nach einem der vorhergehenden Ansprüche, bei dem die organische reduzierend wirkende Verbindung aus Glucose, Glucose-6-phosphat und Isopropanol ausgewählt wird.

6. Verfahren nach einem der vorhergehenden Ansprüche, bei dem die Aminosäure mit einer Thiolgruppe bzw. das Peptid mit einer Thiolgruppe aus Cystein, Homocystein, Glutathion und Thioredoxin ausgewählt wird.

7. Verfahren nach einem der vorhergehenden Ansprüche, bei dem die Vorstufe von L-Methionin aus O-Acetyl-L-homoserin und O-Succinyl-L-homoserin ausgewählt wird.

8. Verfahren nach einem der vorhergehenden Ansprüche, bei dem das Methylmercaptan beim Austritt aus dem Reaktor direkt bei der Synthese des L-Methionins verwendet wird.

9. Verfahren nach Anspruch 8, das mindestens folgende Schritte umfasst:
Schritt 1: Herstellen einer Vorstufe von L-Methionin, beispielsweise durch bakterielle fermentation von Glucose,
Schritt 2: enzymatische Reduktion von DMDS in einem Reaktor R1 unter Bildung von Methylmercaptan, das aus dem Reaktor R1 austritt,
Schritt 3: enzymatische Synthese von L-Methionin in einem Reaktor R2 mit der Vorstufe aus Schritt 1 und
dem Methylmercaptan aus Schritt 2,
Schritt 4 (fakultativ): Rezyklieren des in Schritt 3 gebildeten Gluconolactons zu Schritt 1,
Schritt 5: Gewinnen und gegebenenfalls Reinigen des gebildeten L-Methionins.

10. Verfahren nach einem der Ansprüche 1 bis 7, bei dem die Synthese von Methylmercaptan aus DMDS und die Synthese von L-Methionin aus dem Methylmercaptan in demselben Reaktor durchgeführt werden.

11. Verfahren nach Anspruch 10, das mindestens folgende Schritte umfasst:
Schritt 1' : Herstellen einer Vorstufe von L-Methionin durch bakterielle Fermentation von Glucose,
Schritt 2': enzymatische Reduktion von DMDS in einem Reaktor R1 unter Bildung von Methylmercaptan in situ und gleichzeitige enzymatische Synthese von L-Methionin in demselben Reaktor mit der in Schritt 1' erhaltenen Vorstufe,
Schritt 3' (fakultativ): Rezyklieren des in Schritt 2' gebildeten Gluconolactons zu Schritt 1' und
Schritt 4': Gewinnen und gegebenenfalls Reinigen des gebildeten L-Methionins.

12. Verfahren nach einem der vorhergehenden Ansprüche, das diskontinuierlich oder kontinuierlich durchgeführt wird.

13. Verfahren nach einem der vorhergehenden Ansprüche, bei dem das ideale Molverhältnis von organischer reduzierend wirkender Verbindung zu DMDS von 0,01 bis 100 variiert, das Molverhältnis vorzugsweise zwischen 0,5 und 5 gewählt wird und das Molverhältnis ganz besonders bevorzugt gleich 1 ist.

14. Verfahren nach einem der vorhergehenden Ansprüche, bei dem das Molverhältnis von DMDS zu Vorstufe von L-Methionin zwischen 0,1 und 10, im Allgemeinen zwischen 0,5 und 5, liegt und das Molverhältnis vorzugsweise stöchiometrisch ist (Molverhältnis = 0,5) .

15. Verfahren nach einem der vorhergehenden Ansprüche, bei dem die Temperatur der Reaktion in einem Bereich von 10 °C bis 50 °C, vorzugsweise von 15 °C bis 45 °C und noch weiter bevorzugt von 20 °C bis 40 °C liegt.

## Claims

1. Process for the preparation of L-methionine, comprising at least the steps of:
a) preparation of a mixture comprising:
1) dimethyl disulfide (DMDS),
2) a catalytic amount of an amino acid carrying a thiol group or of a peptide comprising a thiol group,
3) a catalytic amount of enzyme which catalyses the reaction for the reduction of the disulfide bridge of the said amino acid carrying a thiol group or of the said peptide comprising a thiol group,
4) a reducing organic compound in stoichiometric amount with respect to the disulfide, in particular DMDS,
5) a catalytic amount of enzyme which catalyses the reaction for the dehydrogenation of the concerned reducing organic compound,
6) a catalytic amount of a cofactor common to the two enzymes of the catalytic system (dehydrogenase and reductase),
b) carrying out the enzymatic reaction in order to form methyl mercaptan (CH₃-SH),
c) addition of a precursor of L-methionine and conversion of the said precursor with the methyl mercaptan formed in step b), and
d) recovery and optionally purification of the L-methionine formed.

2. Process according to Claim 1, comprising at least the steps of:
a') preparation of a mixture comprising:
• dimethyl disulfide (DMDS),
• a catalytic amount of amino acid carrying a thiol group or of a peptide comprising a thiol group,
• a catalytic amount of reductase enzyme corresponding to the said amino acid carrying a thiol group or to the said peptide comprising a thiol group,
• a catalytic amount of NADPH,
b') addition of a reducing organic compound in stoichiometric amount with respect to the dimethyl disulfide with a catalytic amount of the corresponding dehydrogenase enzyme,
c') carrying out the enzymatic reaction in order to form methyl mercaptan (CH₃-SH),
d') conversion of a precursor of L-methionine with the methyl mercaptan formed in step c'), and
e') recovery and optionally purification of the L-methionine formed.

3. Process according to Claim 1 or Claim 2, in which the methyl mercaptan is brought directly into contact with a methionine precursor.

4. Process according to any one of the preceding claims, in which the reducing organic compound is a hydrogen-reducing organic compound carrying the hydroxyl functional group, chosen from alcohols, polyols or sugars.

5. Process according to any one of the preceding claims, in which the reducing organic compound is chosen from glucose, glucose 6-phosphate and isopropanol.

6. Process according to any one of the preceding claims, in which the amino acid carrying a thiol group or the peptide carrying a thiol group is chosen from cysteine, homocysteine, glutathione and thioredoxin.

7. Process according to any one of the preceding claims, in which the precursor of L-methionine is chosen from O-acetyl-L-homoserine and O-succinyl-L-homoserine.

8. Process according to any one of the preceding claims, in which methyl mercaptan is used directly at the outlet of the reactor in the synthesis of L-methionine.

9. Process according to Claim 8, comprising at least the following steps:
Step 1: preparation of a precursor of L-methionine, for example by bacterial fermentation of glucose,
Step 2: enzymatic reduction of DMDS in a reactor R1 with formation of methyl mercaptan which exits from the said reactor R1,
Step 3: enzymatic synthesis of L-methionine in a reactor R2 with the precursor of Step 1 and the methyl mercaptan of Step 2,
Step 4 (optional): recycling of the gluconolactone formed in Step 3 in Step 1,
Step 5: recovery and optionally purification of the L-methionine formed.

10. Process according to any one of Claims 1 to 7, in which the synthesis of methyl mercaptan from DMDS and the synthesis of L-methionine from the said methyl mercaptan are carried out in one and the same reactor.

11. Process according to Claim 10, comprising at least the following steps:
Step 1': preparation of a precursor of L-methionine by bacterial fermentation of glucose,
Step 2' : enzymatic reduction of DMDS in a reactor R1 with *in situ* formation of methyl mercaptan and joint enzymatic synthesis of L-methionine in the same reactor with the precursor obtained in Step 1',
Step 3' (optional): recycling of the gluconolactone formed in Step 2' in Step 1', and
Step 4': recovery and optionally purification of the L-methionine formed.

12. Process according to any one of the preceding claims, carried out batchwise or continuously.

13. Process according to any one of the preceding claims, in which the ideal reducing organic compound/DMDS molar ratio varies from 0.01 to 100, preferably the said molar ratio is chosen between 0.5 and 5 and entirely preferably the molar ratio is equal to 1.

14. Process according to any one of the preceding claims, in which the DMDS/L-methionine precursor molar ratio is between 0.1 and 10, generally between 0.5 and 5, and preferably the molar ratio is the stoichiometry (molar ratio = 0.5).

15. Process according to any one of the preceding claims, in which the temperature of the reaction is within a range extending from 10°C to 50°C, preferably from 15°C to 45°C, more preferably from 20°C to 40°C.
